# EUROPEAN PATENT SPECIFICATION

(11) **EP 4 117 460 B1**
(45) Date of publication and mention of the grant of the patent: **13.03.2024**
(21) Application number: 21810370.3
(22) Date of filing: 18.11.2021
(51) Int. Cl.: A24B 13/00, D04H 1/64, D04H 1/46, A61K 9/00

(54) **CHEWABLE PRODUCT FOR ORAL DELIVERY OF A SUBSTANCE, AND METHOD OF MANUFACTURING THE SAME**
KAUBARES PRODUKT ZUR ORALEN VERABREICHUNG EINER SUBSTANZ UND VERFAHREN ZUR HERSTELLUNG DAVON
PRODUIT À MÂCHER POUR L'ADMINISTRATION ORALE D'UNE SUBSTANCE ET PROCÉDÉ DE FABRICATION DE CELUI-CI

(30) Priority: 27.11.2020 GB 202018694
(43) Date of publication of application: 18.01.2023
(73) Proprietor: Nonwovenn Ltd, Bridgwater, Somerset TA6 4NZ (GB)
(72) Inventor: MELLIN, Thomas, 62154 VISBY (SE); GENTILCORE, Giovanni, Bath Road Bridgwater Somerset TA6 4NZ (GB); HILL, Dave, Bath Road Bridgwater Somerset TA6 4NZ (GB)
(74) Representative: Mewburn Ellis LLP
(86) International application number: PCT/EP2021/082091
(87) International publication number: WO 2022/112085

(56) References cited:
- WO-A1-2016/090075
- WO-A1-2021/116920
- WO-A2-2012/019025
- US-A1- 2013 149 254
- US-A1- 2016 192 703
- US-A1- 2018 153 211

## Description

### TECHNICAL FIELD

The invention relates to a chewable product, e.g. a chewable dosage form such as a lozenge, tablet, pastille or the like, for oral delivery of a substance. In particular, the invention relates to a chewable product formed from a nonwoven fabric, and a method of manufacturing such a chewable product.

### BACKGROUND TO THE INVENTION

It is known to use such nonwoven fabrics to manufacture products for oral delivery of substances. For example, nonwoven fabrics are used to make pouches for containing an individual portion of a product, such as smokeless tobacco (also known as "snus"), coffee, tea, etc., from which flavour is to be extracted. Examples of oral pouched products formed from a nonwoven fabric can be found in US 2014/0026912 A1 and US 2012/0103353 A1.

Nonwoven fabrics are typically produced using one of three processes: dry-laid, wet-laid or spun melt. Each process entangles fibres or filaments into a web in a manner that does not require weaving or knitting.

A dry-laid process typically comprises forming a loose web of staple fibres. Such webs are subsequently bonded together to create the fabric. The web may be formed by an air laying process, whereby the fibres are randomly orientated in the web. Alternatively, forming the web may include carding the fibres, which aligns their orientation. The air laid or carded web can be bonded using mechanical (e.g. hydroentanglement or needle punching), thermal (e.g. where the web includes thermoplastic fibres) or chemical techniques (e.g. using an adhesive binder), or a combination thereof.

A wet-laid process typically comprises forming a slurry of fibres in water or other suitable liquid, which is deposited on a screen or mesh and then dried to form the web.

A spun melt process typically forms a web from continuous filaments spun directly from liquid (i.e. melted) plastic materials.

Typically, the nonwoven fabrics used to produce pouched products are water-permeable, in order to permit substances (e.g. flavour) from the contents of the pouch to flow out.

Nonwoven fabric may be used to manufacture chewable pouches. For example, US 2018/0153211 A1 discloses a nonwoven fabric for manufacturing a pouched product, in which the pouch includes an elastic mesh of thermoplastic polyamide that ensures the pouch can endure repeated deformations caused by chewing. The elastic mesh in this example has a high percentage of open area (i.e. high porosity) to enable a rapid release rate of flavour from the pouch.

US 2013/0149254 A1 discloses a perforated chewable pouch made of a food grade material selected from silicone, latex, rubber or plastic. The pouch encloses a product that can be in a gel, semi-liquid, and/or liquid form. A user chews, sucks, and/or manipulates the pouch to cause the enclosed flavour product to leach out of the perforations into the user's mouth.

US 2014/0261480 A1 discloses a pouch formed from a fabric comprising melt-blown polymer fibres having a hydrophilic surface coating. The melt-blown material can be an elastomer (e.g. polymeric polyurethane) so that the pouch can tolerate being chewed.

WO 2012/019025 A2 discloses a fabric having tobacco entangled with structural fibers. A described smokeless tobacco product includes smokeless tobacco and structural fibers. The structural fibers form a network in which the smokeless tobacco is entangled. The structural fibers have a composition different from the smokeless tobacco. The tobacco -entangled fabric can have an overall oven volatiles content of at least 10 weight percent. In some described examples, the structural fibers form a nonwoven network. In some described examples, fibrous structures of the smokeless tobacco are entangled with the structural fibers.

US 2016/0172703 A1 discloses oral pouch products. A pouched product configured for insertion into the mouth of a user of that product is described therein. The pouched product can include an outer water-permeable pouch defining a cavity containing a composition adapted for oral use and having a surface area, wherein the outer water-permeable pouch can include a nonwoven spunlaid web comprising a plurality of continuous filament heat sealable fibers.

WO 2016/090075 A1 discloses a smokeless tobacco pouch. A pouched product adapted for release of a releasable component therefrom is described therein. The pouched product can include a water-permeable fabric pouch formed so as to define a cavity therein, a composition contained within the cavity of the water-permeable fabric pouch, the composition comprising one or more releasable components that are released from the composition under mouth conditions and that are capable of movement through the water-permeable fabric pouch, and a release modifying agent adapted to react with at least one of the one or more releasable components in the composition and thereby modify the release thereof from the water-permeable fabric pouch.

### SUMMARY OF THE INVENTION

At its most general, the present invention provides a chewable product for oral delivery of a substance, wherein the chewable product is an essentially homogenous portion of nonwoven fabric impregnated with the substance.

The invention is set out in the appended set of claims.

According to one aspect of the invention, there is provided a chewable product for oral delivery of a substance, the chewable product comprising: a dry-laid needled nonwoven fabric substrate having a matrix of interleaved fibres, wherein the nonwoven fabric substrate has a thickness of 1-2 mm; and a substance held within the matrix, wherein the substance is releasable from the matrix by a chewing action. The nonwoven fabric substrate thus provides a frame for carrying the substance that is both resistant to the chewing action (i.e. does not disintegrate when chewed, bitten or sucked) and capable of releasing the substance. A nonwoven substrate may have an advantage of providing a pleasant or satisfactory mouthfeel.

Unlike the pouch-type products known in the art, the chewable product may be formed as a single piece with the substance distributed in or on it. For example, the substance may be substantially homogenously distributed throughout the matrix. This may be advantageous because it obviates the need for a pouching process, which in turn means that the fabric need not have certain properties, e.g. in relation to air permeability and heat sealability, that are needed for typical pouching processes. Consequently, a wider variety of fibre types may be used for the chewable fabric of the invention compared with a pouch fabric, which in turn may assist in providing a chewable product with desirable mouthfeel and strength.

The substance may be connected (e.g. bonded or otherwise adhered) to fibres in the matrix by a binder. In some examples, the binder comprises a food-grade substance, i.e. a substance that complies with regulations on food contact. Such substances include vinyl acrylic copolymer, vinyl acetate ethylene copolymer and a vinyl acetate copolymer. The binder may also preferably include a biodegradable material such as a polylactic acid (PLA) emulsion or a poly(butylene succinate) (PBS) emulsion.

The nonwoven fabric substrate may comprise staple fibres of a material that complies with food contact regulations, i.e. is approved for use in food packaging or the like. The stable fibres may be natural or regenerated cellulosic fibres such as viscose and/or lyocell. In one example, the nonwoven fabric substrate may comprise a mixture of staple fibres of viscose and thermoplastic fibres. The thermoplastic fibres may comprise 10 to 50 wt% of the substrate, preferably 10 to 30 wt%. This mixture may be advantageous in that provides a desirable balance between strength of the resulting fabric whilst also provided a soft mouthfeel. The thermoplastic fibres may consist of any one or more of: polypropylene, polyethylene, polyester, ethylene vinyl acetate, polyurethane, polylactic acid and polyamides. These materials are preferred because they comply with food contact regulations and are hence suitable for products intended for intra-oral use.

The nonwoven fabric substrate may have a weight equal to or greater than 100 g/m², e.g. in the range 100-500 g/m². Preferably, the nonwoven fabric substrate has a weight equal to or greater than 150 g/m², e.g. in the range of 150-300 g/m². The weight of the fabric substrate provides structural integrity to the chewable product.

It is desirable for the nonwoven fabric substrate to have a tensile strength that can withstand the forces associated with chewing. It may be particularly important for the nonwoven fabric substrate to have a tensile strength above a predetermined threshold when in a wet state, e.g. saturated with water or the like. The tensile strength when in a wet state may be referred to herein as a "wet strength" of the nonwoven fabric substrate. The fibres in the nonwoven fabric substrate may be generally aligned in a first direction (referred to herein as the machine direction) that is perpendicular to a second direction (referred to herein as the cross direction). A wet strength of the nonwoven fabric substrate may be defined in each of the machine direction and the cross direction. In one example, the nonwoven fabric substrate preferably has a wet strength that is equal to or greater than 5 N/cm in the cross direction and/or 30 N/cm in the machine direction. The tensile strength of the nonwoven fabric substrate when in a dry state may be equal to or greater than 15 N/cm in the cross direction and equal to or greater than 15 N/cm in the machine direction.

The tensile strength of a nonwoven (both in a dry state and in a wet state) may be measured using the grab strength test method published as NWSP 110.1 by EDANA.

It is also desirable for the chewable product to have soft mouth feel for the user. Soft mouth feel may be achieved by ensuring that the surface of the nonwoven fabric substrate has a non-abrasive characteristic. For example, interferometric techniques may be used to measure a surface texture of the nonwoven fabric substrate.

The matrix of interleaved fibres is formed by needle punching. The nonwoven fabric substrate thus comprises a precursor web that is subjected to needle punching to obtain a needle-punched fabric.

Needle punching is a mechanical web consolidation technique in which an array of needles are reciprocally inserted and withdrawn into a web of fibres, usually in a direction perpendicular to a plane of the web. Needle punching may be a particularly useful technique for physical consolidation because it is capable of producing homogenous fabrics with high tensile strength in three dimensions, i.e. the machine direction, the cross direction and a height direction (also referred to as "loft") of the fabric. Moreover, through appropriate selection of the needle punching parameters, the loft, strength and density of the nonwoven fabric substrate may be controlled in a manner that enables repeatable consistent manufacture of the chewable product.

The needle punching parameters may comprise any one or more of (i) needle geometry, (ii) needle density (i.e. the spacing between needles in the array of needles, and (iii) speed of perforation.

Relevant features of the needle geometry may comprise the size of the needle, e.g. length and outer diameter. The length may be defined in terms of penetration distance into the web. In some examples, the penetration distance may be equal to or greater than 10 mm, e.g. in the range 10-17 mm. The outer diameter may be defined by a wire gauge. For example, each needle may have a wire gauge in the range 32-38, i.e. an outer diameter in the range 0.1-0.2 mm. The needle may also comprise surface features, e.g. a barb or the like, to facilitate fibre entanglement.

The array of needles (which may also be referred to as a needle loom) may comprises a line of needles spaced in the cross direction. Such an array of needles preferably has a density equal to or greater than 400 needles/m, e.g. in the range 400-6000 needles/m.

The needle density influences the density of perforations or "stitches" formed in the web. The other factors that affect the stitch density are the speed of the web through a needle punching area and the stroke rate of the needle array, which corresponds to the speed of perforation. The speed of the web may be controllable at both an input to and an output from the needle punching area. In one example, the input and output speeds may be equal. In another example, the output speed may be less than the input speed, to promote entanglement and increasing fibre consolidation in the machine direction. Both the input and output speeds may be in the range 2-7 m/min. The stroke rate of the needle array is preferably equal to or greater than 180 rpm, e.g. in the range 180-750 rpm.

The needle punching area may be defined by a stripper plate, which may be a planar element that lies over the web to hold the web in place while the needles repeatedly penetrate it. The stripper plate may have a length in the machine direction equal to or greater than 10 mm, e.g. in the range 10-20 mm.

The substance that is held within the matrix of interleaved fibres may comprise a carrier medium and an active agent. The substance is desirable removable from the matrix under a chewing action. The carrier medium may be configured to facilitate the releasably retention of the substance to the fibres in the matrix. In some examples, the carrier medium may be the same material as the binder mentioned above.

Alternatively, the carrier medium may comprise a diffusion restricting material that inhibits diffusion of liquid into the nonwoven fabric substrate, and hence facilitates retention of the active agent in the matrix until the product is subjected to a chewing action in an aqueous environment. In one example, the diffusion restricting material may comprise a digestible liquid-impermeable component. The digestible liquid-impermeable component may comprise or consist of any one or any combination of: a modified starch, hydroxypropyl methylcellulose (HPMC), polyvinyl alcohol (PVA), lactose and sucrose.

The diffusion restricting material may comprise a superabsorbent polymer. The superabsorbent polymer may operate to absorb water and swell to create a physical barrier to diffusion within the nonwoven fabric substrate. The physical barrier may be overcome by chewing. The superabsorbent polymer may be water soluble, so that it is gradually removed when in an aqueous environment. The superabsorbent polymer may comprise or consist of any one or any combination of: a water-soluble grade of sodium polyacrylate; a superabsorbent alginate; and carboxymethyl cellulose.

The diffusion restricting material may comprise a hydrophobic material. The hydrophobic material may comprise or consist of any one or any combination of: polyethylene; polytetrafluoroethylene; and a fluorosurfactant.

The substance (or active agent thereof) may comprise any of smokeless tobacco, nicotine salts, cannabidiol, coffee, tea, flavouring or the like. The substance may further comprise one or more media configured to remove impurities, e.g. active charcoal or zeolites. In one example, the substance may comprise a pharmaceutical material such as aspirin, paracetamol and ibuprofen. The chewable product may therefore comprise a dosage form for the delivery of medication. The nonwoven substrate may be shaped to resemble conventional oral dosage forms, e.g. as a tablet, pill or lozenge. The chewable product may resemble a miniature pillow, e.g. having a cuboidal shape. The nonwoven substrate has a thickness of 1-2 mm.

The chewable product may further comprise packaging for sealably containing the nonwoven fabric substrate. The packaging may comprise any one of: a foil wrapper, a blister pack, a sealable container.

In another aspect, there is provided a method of manufacturing a chewable product for oral delivery of a substance, the method comprising: forming an initial web of dry-laid carded nonwoven fibres; passing the initial web through a needle loom to form a matrix of interleaved fibres; applying a substance to the web using any of a roll-to-roll coating process, a printing process, a laminator, a dip-coating unit or a spray unit, whereby the substance is held within the matrix and releasable therefrom by a chewing action; and cutting the web into a plurality of individual portions.

Features of the first aspect discussed above may be equally applicable to the second aspect. The carding and needling of the web may be configured to produce a nonwoven fabric substrate that possesses structural integrity to be resistant to a chewing action, i.e. to avoid disintegration when bitten, sucked or otherwise deformed in an aqueous environment. The nonwoven fabric substrate itself provides the matrix, which is a framework for supporting the substance to be delivered.

In one example, the nonwoven fibres may comprise a mixture of staple fibres of viscose and thermoplastic fibres. The method may further comprise heating the matrix of interleaved fibres. The heating step acts to improve or strengthen links between the thermoplastic fibres and other fibres in the matrix.

The substance is applied using any of a roll-to-roll coating process, a printing process, a laminator, a dip-coating unit or a spray unit. For example, the step of applying the substance may comprise dipping the matrix of interleaved fibres in a liquid containing the substance. The method may include a step of removing any excess liquid, e.g. by rolling and/or drying the matrix. In another example, applying the substance may comprise spraying the matrix with a liquid or powder containing the substance.

The substance may comprise a carrier medium and an active agent, as discussed above. In this case, the step of applying the substance may comprise binding the carrier medium to the matrix of interleaved fibres.

The method may further comprise packaging the plurality of individual portions, e.g. by individual wrapping each of the plurality of individual portions.

### BRIEF DESCRIPTION OF THE DRAWINGS

Embodiments of the invention is discussed below in more detail with reference to the accompanying drawings, in which:
Fig. 1 is a schematic drawing of apparatus for manufacture of a chewable product according to a method that is an embodiment of the invention;
Fig. 2 is a schematic drawing of a chewable product that is an embodiment of the invention; and
Fig. 3 is a flowchart of a method for manufacturing of a chewable product that is an embodiment of the invention.

### DETAILED DESCRIPTION; FURTHER OPTIONS AND PREFERENCES

Discussed in detail below is apparatus and method for manufacturing a chewable product from nonwoven fabric that has a substance applied in or on it, wherein that substance is releasable from the nonwoven fabric by a chewing action when in a user's mouth.

Herein, the term "chewing action" may mean any one or any combination of (1) presence of saliva, (2) presence of water, and (3) mechanical deformation. The ability to release the substance from the chewable product may arise any or any combination of this conditions.

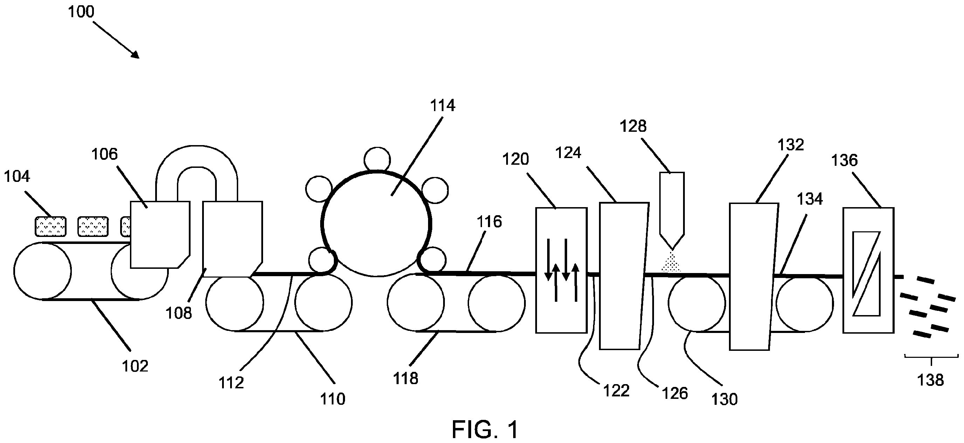

Fig. 1 is a schematic diagram showing an apparatus 100 for manufacturing a chewable product that is an embodiment of the invention. The chewable product is formed from a nonwoven fabric, which in Fig. 1 is fabricated using a dry-laid carded technique. However, it is to be understood that the invention may be used with nonwoven fabrics obtained using other types of manufacturing technique, including wet-laid or spun melt techniques.

In the apparatus 100 shown in Fig. 1, a first conveyor 102 transports fibre bales 104 to a bale opener 106, which separates and blends the fibres from each bale. The fibre bales 104 may comprises bundles of any suitable staple fibre, such as regenerated cellulose (viscose) or the like. The bale opener 106 is connected to a feed hopper 108 that discharges the blended fibres as a loose fibre web 112 on a second conveyor 110. The loose fibre web 112 is conveyed to a carding machine 114 that combs the web to apply a desired orientation or plurality of orientations to the fibres in the web. The carding machine 114 thus outputs a carded web 116 on to a third conveyor 118.

In this example, the carded web 116 is then further consolidated by passing through a needle loom 120. The needle loom 120 is configured to perform needle punching on the carded web 116 in order to increase its structural integrity. The needle punching cause the fibres within the carded web 116 to become physically entangled, which increases the strength of the carded web 116.

In this example, the needle loom 120 is configured to output a needled web 122 having a weight greater than 100 g/m². In one example, the needle loom 120 may comprise a plurality of needles each having a wire gauge in the range 32-38, arranged on the loom with a density equal to or greater than 400 needles/m.

The carded web 116 is fed into the needle loom 120 at an input speed, which is preferably in the range 2-7 m/min. The stroke rate of the needle array is controlled in conjunction with the speed of the web through the needle loom and the needle density to obtain the desired stitch density.

The needle loom 120 outputs a needled web 122. The needled web 122 may already be strong enough to form the chewable product. In one example, the needled web 122 may be rolled or otherwise gathered for transport to another location for the remaining steps in the process of forming the chewable product. In other examples, the needled web 122 may be subject to one or more further strengthening treatments. This may be done, for example, if the fibres in the needled web 122 comprise thermoplastic fibres. As shown in Fig. 1, for example, the needled web 122 may be heated within a heater 124 or by passing the needled web 122 through heated calendar rollers to create or strengthen bonds between the thermoplastic fibres and the rest of the fibre matrix. As mentioned above, the thermoplastic fibres may consist of any one or more of: polypropylene, polyethylene, polyester, ethylene vinyl acetate, polyurethane, polylactic acid and polyamides.

Heating the needled web 122 may act to further consolidate the fabric, e.g. by stiffening or increasing its density. The parameters of the heating step can be selected to ensure that the resulting fabric has a desired loft for use in the chewable product.

Following the needle punching process and the optional heating process, the nonwoven fabric is a precursor web 126 ready to receive a substance that is to be delivered by the chewable product.

The substance may be applied by any suitable means. Preferably, the substance is applied in a manner that causes it to permeate the precursor web 126 such that the resulting product has an essentially homogeneous distribution of the substance within it.

The substance may be applied in aqueous form, e.g. by dipping or spraying the precursor web 126 so that the substance impregnates or saturates the precursor web 126. Alternatively, the substance may be applied in the form of a powder, e.g. a dry granular material, which can be applied as a spray or via a roller. Fig. 1 illustrate a spray applicator 128 by way of example.

In some examples, the substance may be combined with a carrier or other excipient when applied to the nonwoven material. The carrier may be selected to assist attachment to the nonwoven material. For example, it may be a material that acts to bind to the nonwoven fibres. The binder may comprise a substance that complies with regulations on food contact. Such substances include vinyl acrylic copolymer, vinyl acetate ethylene copolymer and a vinyl acetate copolymer. The binder may also preferably consist or comprise a biodegradable material such as a polylactic acid (PLA) emulsion or a poly(butylene succinate) (PBS) emulsion.

In other examples, the substance may include an active agent (e.g. a pharmaceutical or the like), and the excipient may act as both a carrier for that active agent and as a means to assist in distributing it through the nonwoven matrix.

The sprayed web is then transported to a fourth conveyor 130 optionally via nip rollers (not shown), which may operate to remove or squeeze any excess liquid from the web. The sprayed web is then carried through a dryer 132.

The resulting fabric 134 may then be ready for final forming and packaging as the chewable product. These steps may be performed in the same or a different location to the manufacture of the nonwoven web and/or application of the substance. For example, after exiting the dryer 132, the resulting fabric 134 may be wound into a finished roll for transport elsewhere. The fabric 134 may also be subjected to other finishing processes, e.g. applying pigment or dye, or printing on to the fabric.

In Fig. 1, the resulting fabric 134 is conveying to a cutting device 136 that operates to divide the sheet-like fabric into a plurality of individual portions 138, each of which form a chewable product. Each individual portion may resemble a lozenge, tablet or pastille. Any suitable sheet cutting machine may be used for this purpose.

The plurality of portions 138 may subsequently be packaged. For example, each individual portion may be hermetically sealed, e.g. in a foil casing or blister pack.

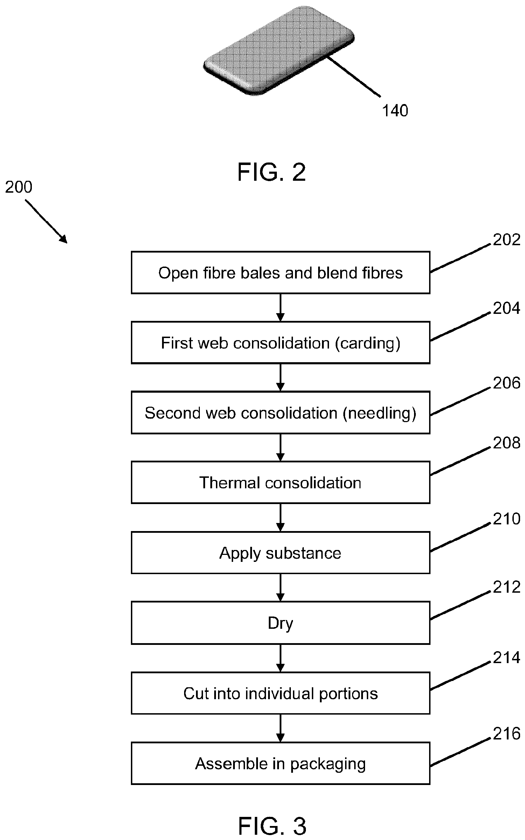

Fig. 2 illustrates one example of a chewable product 140 that is an embodiment of the invention. The chewable product 140 in this example takes the form of a cuboidal lozenge, but it may be understood that other shapes can be used, e.g. cylindrical pill, disc-shaped tablet, etc. The chewable product 140 preferably resembles a conventional pastille or tablet.

Fig. 3 is a flowchart of a method 200 for manufacturing of a chewable product that is an embodiment of the invention. The method 200 may use the apparatus discussed above in relation to Fig. 1.

The method 200 begins with a step 202 of opening fibre bales and blending fibres with which a main substrate or matrix of the chewable product is to be formed. As explained above, the fibres may comprise regenerated cellulose (viscose) fibres, and may optionally include thermoplastic fibres (e.g. polypropylene or the like), e.g. in a proportion in the range 10 to 50 wt% of the blended fibres. The blended fibres are deposited in a loose layer.

The method 200 continues with a first web consolidation step 204, which in this example comprises carding the layer of blended fibres. The method 200 continues with a second web consolidation step 206, which in this example comprises needle punching the carded web in order to further interlink and strengthen the web, thereby making it resistant to break up under the types of mechanical deformation associated with the chewing, sucking, biting or the like.

The method 200 continues with a thermal consolidation step 208 in which the needled web is heated. This step may be optional. For example it may only be performed if the blended fibres include thermoplastic fibres. Heating the web may strengthen bonds between the thermoplastic fibres within the web, thereby further strengthening the fabric matrix.

The method 200 continues with a step 210 of applying the substance to be delivered when a chewable product formed from the nonwoven material is used. As mentioned above, the substance may be applied directly or together with a carrier medium, excipient or binder. The applying step may comprise impregnating the fibre matrix with a solution (e.g. aqueous solution) or slurry of the substance. The fibre matrix may be dipped, sprayed or otherwise coated in order to apply the substance.

The method 200 continues with a step 212 of drying the nonwoven material. The drying may be to drive off any remaining carrier medium (e.g. water) with which the substance was applied. Alternatively or additionally, the drying step may serve to 'cure' or set a binder that acts to secure the substance within the nonwoven fibre matrix.

The method 200 continues with a step 214 of cutting the dried sheet of nonwoven fabric containing the substance into individual portions, e.g. having desired dimensions for using as a chewable product. The method 200 continues with a step 216 of packaging the individual portions. The packaging step 216 may comprises individually wrapping each individual portion, e.g. in a foil wrapper or blister pack. Alternatively the packaging step may comprises filling sealable containers with a plurality of individual portions.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A chewable product for oral delivery of a substance, the chewable product comprising:
a dry-laid needled nonwoven fabric substrate having a matrix of interleaved fibres, wherein the nonwoven fabric substrate has a thickness of 1-2 mm; and
a substance held within the matrix,
wherein the substance is releasable from the matrix by a chewing action.

2. The chewable product of claim 1, wherein the substance is substantially homogenously distributed throughout the matrix.

3. The chewable product of claim 1 or 2, wherein the substance is connected to fibres in the matrix by a binder.

4. The chewable product of claim 3, wherein the binder comprises any of vinyl acrylic copolymer, vinyl acetate ethylene copolymer, a vinyl acetate copolymer, polylactic acid (PLA) or poly(butylene succinate) (PBS).

5. The chewable product of any preceding claim, wherein the nonwoven fabric substrate comprises staple fibres of viscose, or comprises a mixture of staple fibres of viscose and thermoplastic fibres.

6. The chewable product of any preceding claim, wherein the nonwoven fabric substrate has a weight equal to or greater than 100 g/m².

7. The chewable product of any preceding claim, wherein the substance comprises a pharmaceutical.

8. The chewable product of any preceding claim further comprising packaging for sealably containing the nonwoven fabric substrate.

9. A method of manufacturing a chewable product for oral delivery of a substance, the method comprising:
forming an initial web of dry-laid carded nonwoven fibres;
passing the initial web through a needle loom to form a matrix of interleaved fibres;
applying a substance to the web using any of a roll-to-roll coating process, a printing process, a laminator, a dip-coating unit or a spray unit, whereby the substance is held within the matrix and releasable therefrom by a chewing action; and
cutting the web into a plurality of individual portions.

10. The method of claim 9, wherein the step of forming the initial web comprises depositing the nonwoven fibres with a weight equal to or greater than 100 g/m².

11. The method of claim 9 or 10, wherein passing the initial web through a needle loom comprising penetrating the web with an array of needles at a stroke rate equal to or greater than 180 rpm.

12. The method of claim 11, wherein the array of needles has a needle density equal to or greater than 400 needles/m.

13. The method of any one of claims 9 to 12, wherein the web passes through the needle loom at a speed in the range 2-7 m/min.

14. The method of any one of claims 9 to 13, wherein the nonwoven fibres comprise a mixture of staple fibres of viscose and thermoplastic fibres, and wherein the method further comprises heating the matrix of interleaved fibres.

15. The method of any one of claims 9 to 14 further comprising packaging the plurality of individual portions by individually wrapping each of the plurality of individual portions.

## Patentansprüche

1. Kaubares Produkt zur oralen Zufuhr einer Substanz, wobei das kaubare Produkt Folgendes umfasst:
ein trockengelegtes genähtes Vliesstoffsubstrat, das eine Matrix aus überlappenden Fasern aufweist, wobei das Vliesstoffsubstrat eine Dicke von 1 bis 2 mm aufweist; und
eine Substanz, die innerhalb der Matrix gehalten wird,
wobei die Substanz durch eine Kaubewegung aus der Matrix freisetzbar ist.

2. Kaubares Produkt nach Anspruch 1, wobei die Substanz im Wesentlichen homogen in der Matrix verteilt ist.

3. Kaubares Produkt nach Anspruch 1 oder 2, wobei die Substanz durch ein Bindemittel mit den Fasern in der Matrix verbunden ist.

4. Kaubares Produkt nach Anspruch 3, wobei das Bindemittel ein beliebiges aus Vinyl-Acryl-Copolymer, Vinylacetat-Ethylen-Copolymer, einem Vinylacetat-Copolymer, Polymilchsäure (PLA) oder Poly(butylensuccinat) (PBS) umfasst.

5. Kaubares Produkt nach einem der vorangegangenen Ansprüche, wobei das Vliesstoffsubstrat Stapelfasern aus Viskose umfasst oder ein Gemisch aus Stapelfasern aus Viskose und thermoplastischen Fasern umfasst.

6. Kaubares Produkt nach einem der vorangegangenen Ansprüche, wobei das Vliesstoffsubstrat ein Gewicht größer oder gleich 100 g/m² aufweist.

7. Kaubares Produkt nach einem der vorangegangenen Ansprüche, wobei die Substanz ein Pharmazeutikum umfasst.

8. Kaubares Produkt nach einem der vorangegangenen Ansprüche, das weiters eine Verpackung zum dichten Enthalten des Vliesstoffsubstrats umfasst.

9. Verfahren zur Herstellung eines kaubaren Produkts zur oralen Zufuhr einer Substanz, wobei das Verfahren Folgendes umfasst:
Bilden eines anfänglichen Netzes aus trockengelegten gekardeten Vliesfasern;
Führen des anfänglichen Netzes durch einen Nadelwebstuhl, um eine Matrix aus überlappten Fasern zu bilden;
Aufbringen einer Substanz auf das Netz unter Verwendung eines beliebigen aus einem Walze-auf-Walze-Beschichtungsvorgangs, eines Druckvorgangs, eines Laminators, einer Tauchbeschichtungseinheit und/oder einer Sprüheinheit, wodurch die Substanz innerhalb der Matrix gehalten wird und durch eine Kauhandlung daraus freisetzbar ist; und
Schneiden des Netzes in eine Vielzahl einzelner Abschnitte.

10. Verfahren nach Anspruch 9, wobei der Schritt des Bildens des anfänglichen Netzes das Ablagern der Vliesstofffasern mit einem Gewicht von größer oder gleich 100 g/m² umfasst.

11. Verfahren nach Anspruch 9 oder 10, wobei das Führen des anfänglichen Netzes durch einen Nadelwebstuhl das Durchdringen des Netzes mit einer Anordnung von Nadeln mit einer Stoßrate von größer oder gleich 180 rpm umfasst.

12. Verfahren nach Anspruch 11, wobei die Anordnung von Nadeln eine Nadeldichte von größer oder gleich 400 Nadeln/m aufweist.

13. Verfahren nach einem der Ansprüche 9 bis 12, wobei das Netz mit einer Geschwindigkeit im Bereich von 2 bis 7 m/min durch den Nadelwebstuhl geführt wird.

14. Verfahren nach einem der Ansprüche 9 bis 13, wobei die Vliesstofffasern ein Gemisch aus Stapelfasern aus Viskose und thermoplastischen Fasern umfassen und wobei das Verfahren ferner das Erhitzen der Matrix aus überlappten Fasern umfasst.

15. Verfahren nach einem der Ansprüche 9 bis 14, das ferner das Verpacken der Vielzahl von einzelnen Abschnitten durch einzelnes Einwickeln eines jeden aus der Vielzahl von einzelnen Abschnitten umfasst.

## Revendications

1. Produit à mâcher pour l'administration orale d'une substance, le produit à mâcher comprenant :
un substrat de tissu non tissé aiguilleté par voie sèche présentant une matrice de fibres entrelacées, dans lequel le substrat de tissu non tissé présente une épaisseur de 1 à 2 mm ; et
une substance maintenue à l'intérieur de la matrice,
dans lequel la substance est libérable à partir de la matrice par une action de mastication.

2. Produit à mâcher selon la revendication 1, dans lequel la substance est distribuée de manière sensiblement homogène dans toute la matrice.

3. Produit à mâcher selon la revendication 1 ou 2, dans lequel la substance est reliée à des fibres dans la matrice par un liant.

4. Produit à mâcher selon la revendication 3, dans lequel le liant comprend un quelconque parmi un copolymère de vinyle acrylique, un copolymère d'acétate de vinyle et d'éthylène, un copolymère d'acétate de vinyle, de l'acide polylactique (PLA) ou du poly(succinate de butylène) (PBS).

5. Produit à mâcher selon l'une quelconque des revendications précédentes, dans lequel le substrat de tissu non tissé comprend des fibres discontinues de viscose, ou comprend un mélange de fibres discontinues de viscose et de fibres thermoplastiques.

6. Produit à mâcher selon l'une quelconque des revendications précédentes, dans lequel le substrat de tissu non tissé présente un poids égal ou supérieur à 100 g/m².

7. Produit à mâcher selon l'une quelconque des revendications précédentes, dans lequel la substance comprend un produit pharmaceutique.

8. Produit à mâcher selon l'une quelconque des revendications précédentes, comprenant en outre un emballage pour contenir de manière étanche le substrat de tissu non tissé.

9. Procédé de fabrication d'un produit à mâcher pour l'administration orale d'une substance, le procédé comprenant les étapes consistant à :
former une bande initiale de fibres non tissées cardées par voie sèche ;
faire passer la bande initiale à travers un métier à aiguilles pour former une matrice de fibres entrelacées ;
appliquer une substance sur la bande en utilisant un quelconque parmi processus d'application en revêtement rouleau à rouleau, un processus d'impression, un dispositif de stratification, une unité d'application en revêtement par immersion ou une unité de pulvérisation, la substance étant maintenue à l'intérieur de la matrice et pouvant en être libérée par une action de mastication ; et
découper la bande en une pluralité de parties individuelles.

10. Procédé selon la revendication 9, dans lequel l'étape de formation de la bande initiale comprend le dépôt des fibres non tissées avec un poids égal ou supérieur à 100 g/m².

11. Procédé selon la revendication 9 ou 10, dans lequel l'étape consistant à faire passer la bande initiale à travers un métier à aiguilles comprend l'étape consistant à pénétrer la bande avec un réseau d'aiguilles à une vitesse de course égale ou supérieure à 180 tr/min.

12. Procédé selon la revendication 11, dans lequel le réseau d'aiguilles présente une densité d'aiguilles égale ou supérieure à 400 aiguilles/m.

13. Procédé selon l'une quelconque des revendications 9 à 12, dans lequel la bande passe à travers le métier à aiguilles à une vitesse comprise entre 2 et 7 m/min.

14. Procédé selon l'une quelconque des revendications 9 à 13, dans lequel les fibres non tissées comprennent un mélange de fibres discontinues de viscose et de fibres thermoplastiques, et dans lequel le procédé comprend en outre l'étape consistant à chauffer la matrice de fibres entrelacées.

15. Procédé selon l'une quelconque des revendications 9 à 14, comprenant en outre l'emballage de la pluralité de parties individuelles en enveloppant individuellement chacune de la pluralité de parties individuelles.
